(19) Europäisches Patentamt
European Patent Office
Office européen des brevets

(11) **EP 2 726 576 B1**

(12) **FASCICULE DE BREVET EUROPEEN**

(45) Date de publication et mention de la délivrance du brevet:
**01.06.2022 Bulletin 2022/22**

(21) Numéro de dépôt: **12743480.1**

(22) Date de dépôt: **29.06.2012**

(51) Classification Internationale des Brevets (IPC):
**C10G 11/18** (2006.01)   **B01J 38/30** (2006.01)

(52) Classification Coopérative des Brevets (CPC):
**C10G 11/182; B01J 38/02; B01J 38/30;**
**C10G 11/187;** C10G 2300/1014; C10G 2300/301;
C10G 2300/708; Y02P 30/20

(86) Numéro de dépôt international:
**PCT/FR2012/051505**

(87) Numéro de publication internationale:
**WO 2013/001245 (03.01.2013 Gazette 2013/01)**

(54) **PROCÉDÉ DE CRAQUAGE CATALYTIQUE POUR LE TRAITEMENT D'UNE COUPE À FAIBLE CARBONE CONRADSON**

KATALYTISCHES KRACKVERFAHREN ZUR BEHANDLUNG EINER FRAKTION MIT NIEDRIGEM GEHALT AN CONRADSON-KOHLENSTOFF

CATALYTIC CRACKING METHOD FOR TREATING A FRACTION HAVING A LOW AMOUNT OF CONRADSON CARBON

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.06.2011 FR 1155852**
**08.08.2011 US 201161521101 P**

(43) Date de publication de la demande:
**07.05.2014 Bulletin 2014/19**

(73) Titulaire: **TotalEnergies Raffinage France**
**92400 Courbevoie (FR)**

(72) Inventeurs:
• **BORIES, Marc**
**76280 Saint-Jouin-Bruneval (FR)**

• **LEROY, Patrick**
**76430 Saint-Vigor-d'Ymonville (FR)**
• **GBORDZOE, Eusebius**
**Houston, Texas 77084 (US)**

(74) Mandataire: **Raboin, Jean-Christophe et al**
**Total Research & Technology Feluy**
**Zone Industrielle C**
**7181 Seneffe (BE)**

(56) Documents cités:
**US-A- 2 902 432      US-A- 3 692 667**
**US-A- 4 436 613      US-A- 4 828 680**
**US-A- 4 875 994      US-A- 4 980 050**
**US-A1- 2001 025 806  US-A1- 2008 035 527**

**Description**

La présente invention se situe dans le domaine du craquage catalytique de coupes pétrolières, plus particulièrement de coupes présentant un faible niveau de carbone Conradson et une forte teneur en hydrogène et qui, de ce fait, rendent difficile l'obtention du bilan thermique de l'unité de craquage catalytique (notée FCC).

Dans une unité FCC, le bilan thermique est principalement assuré par la combustion du coke déposé sur le catalyseur lors de l'étape de craquage. La chaleur de combustion permet de chauffer le catalyseur à des températures allant typiquement de 670 à 750°C. Le catalyseur libère au réacteur la chaleur emmagasinée au régénérateur pour vaporiser la charge à craquer injectée sous forme liquide et apporter l'énergie nécessaire pour le craquage de la charge via des réactions globalement endothermiques. On dit du FCC qu'il s'agit d'une unité équilibrée thermiquement car l'énergie produite au régénérateur est alors transportée puis consommée au réacteur par le biais de la circulation de catalyseur. Typiquement, le catalyseur entre dans la zone de régénération avec une teneur en coke (définie comme la masse de coke sur la masse de catalyseur en pourcent poids) comprise entre 0,5 et 1.45 % poids et ressort de la dite zone avec une teneur en coke comprise entre 0.1 et 0.5 % poids pour des régénérateurs fonctionnant en combustion partielle ou comprise entre 0.1 et 0.05 % poids voire inférieure à 0,01 % poids pour des régénérateurs fonctionnant en combustion totale. Lors du fonctionnement d'un régénérateur en combustion partielle ou totale, la combustion est réalisée à l'aide d'un gaz contenant de l'oxygène.

Dans une régénération à combustion totale, l'intégralité du coke est brûlé (teneur typique de CO ou monoxyde de carbone dans les fumées proche de zéro) alors qu'en combustion partielle, la combustion du coke produit du CO à hauteur de quelques pourcents volume, typiquement de 0.5 à 10% volume selon le débit d'air et le degré de d'achèvement de la combustion pour les combustions incomplètes.

La teneur en carbone Conradson (ou CCR) de la charge est définie par la norme ASTM D 482 et représente pour l'Homme du métier une évaluation de la quantité de coke que la charge peut produire au cours de la réaction de craquage catalytique qui a lieu dans le réacteur principal de l'unité. En fonction de la teneur en carbone Conradson de la charge, il est possible de dimensionner l'unité pour un rendement en coke correspondant au craquage de la charge afin de satisfaire le bilan thermique de l'unité qui va commander son bon fonctionnement.

Les coupes lourdes conventionnelles traitées dans une unité de FCC ont généralement des valeurs de carbone Conradson comprises dans la fourchette 0,2 à 10% poids.

Les coupes traitées dans une unité de FCC selon la présente invention peuvent avoir un carbone Conradson inférieur ou égal à 0,1% poids et une teneur en hydrogène supérieure ou égale à 12,7% poids.

EXAMEN DE L'ART ANTERIEUR

Il est connu de l'homme du métier pour équilibrer les bilans thermiques de pousser la combustion au régénérateur en y injectant plus d'air pour une quantité de coke donnée, c'est à dire diminuer le pourcentage volume de monoxyde de carbone (ou CO) dans les fumées, ce qui contribue à augmenter la température audit régénérateur et doit aider à assurer le bilan thermique de l'unité.

Lorsque cette mesure n'est pas suffisante ou possible, il est connu de l'art antérieur de recycler au niveau du régénérateur un combustible sous forme liquide dans un brûleur généralement placé dans la phase dense du lit de catalyseur. Ce combustible, souvent appelé par l'homme du métier combustible à haute viscosité ou encore « torch oil », contient généralement une ou plusieurs coupes « lourdes » dont le point d'ébullition initial est supérieur à 360°C à dominante aromatique. Ces coupes peuvent provenir du FCC ou toute autre unité de conversion dans la raffinerie comme un coker, un viscoréducteur, etc. Il est également possible de recourir à d'autres types de combustible comme du Fioul N°2 ou encore des Fiouls ne répondant pas aux spécifications requises pour leur mise sur le marché. Cette injection de combustible au régénérateur est une pratique usuelle dans les phases de démarrage de l'unité, mais peut être source de problèmes lors d'une utilisation en continu. En effet, comme les températures dans le régénérateur sont de l'ordre de 650°C à 750°C, une partie du recycle se vaporise en formant des gaz craqués qui ne sont pas entièrement oxydés dans la phase dense du régénérateur et vont alors se retrouver dans la phase diluée du régénérateur où ils risquent ainsi de créer des points chauds dommageables au bon fonctionnement du régénérateur. Ce phénomène souvent appelé "afterburning" ou post combustion, peut se définir comme une reprise de combustion en un point non souhaité du régénérateur, par exemple en phase diluée où le catalyseur solide est présent en plus faible quantité, ou en entrée ou à l'intérieur d'un des cyclones également présents dans l'enceinte du régénérateur, ou encore dans les conduites d'évacuation des gaz de combustion. On conservera dans la suite du texte le terme d'"'afterburning" bien admis et pratiqué par l'homme du métier.

Par ailleurs, ce flux de recycle risque de brûler dans le lit de catalyseur en formant localement un front de flamme à haute température. Ce front de flamme est générateur de points chauds avec des températures localement fortes au sein du lit de catalyseur. Comme de la vapeur d'eau se forme également au moment de la combustion de ces hydrocarbures, ces fortes températures locales combinées à la présence de vapeur d'eau fragilisent la partie active du catalyseur (zéolithe) et désactivent ainsi sa fonction craquante. On parle de désactivation hydrothermale du catalyseur. Ainsi plus cette coupe recyclée sera légère, plus elle sera riche en hydrogène, et plus elle génèrera de vapeur d'eau par combustion au niveau du régénérateur. Tous ces phénomènes indésirables décrits précédemment sont évidemment exacerbés au fur et à mesure que la quantité de combustible liquide recyclée au régénérateur pour assurer le bilan thermique de l'unité FCC augmente.

Dans un tel contexte, c'est à dire en présence d'une charge qui produit peu de coke lors de l'opération de craquage, et pour éviter d'introduire en continu dans le régénérateur des hydrocarbures liquides (ou encore « torch oil ») responsables, entre autres, de phénomènes d'afterburning et de la dégradation du catalyseur, les raffineurs ont souvent choisi d'installer un four de préchauffe de la charge utilisant un combustible à teneur élevée en hydrogène en amont du réacteur de craquage. L'ajout d'un tel four permet alors d'apporter les calories qui viennent s'ajouter à celles produites par la combustion du coke sur le catalyseur dans le régénérateur et ainsi équilibrer le bilan thermique de l'unité. L'apport de calories par le four sera d'autant plus élevé que l'apport de calories par le régénérateur sera faible. Il existe cependant une limite de la température de préchauffe maximum pour la charge qui correspond à la température de début de craquage de celle-ci. Soulignons que l'installation de tels fours est coûteuse à l'achat mais aussi à l'utilisation car coûteuse en énergie externe consommée.

Dans un de ses premiers objectifs, la présente invention vise un procédé de craquage catalytique d'une charge peu cokante, présentant un Carbone Conradson inférieur ou égal à 0.1% poids et/ou une teneur en hydrogène supérieure ou égale à 12.7% poids, comprenant l'introduction à l'état divisé dans le lit fluidisé dense du régénérateur constitué de grains de catalyseur plus ou moins cokés d'un matériau riche en coke permettant d'augmenter la quantité de coke à brûler dans le régénérateur sans favoriser la formation de points chauds autour des grains de catalyseur (afterburning) afin d'éviter leur désactivation.

Il est connu d'introduire des composés de type coke au niveau du réacteur de craquage et de les mélanger avec le catalyseur régénéré en une ou plusieurs fois, l'objectif étant de favoriser la dé-métallisation des charges lourdes, les métaux lourds de type nickel et vanadium étant piégés dans les pores du dit coke. De telles applications sont revendiquées dans les brevets US3092568 et US4875994.

Dans le brevet US 4828680, on injecte du coke à l'état divisé dispersé dans du catalyseur frais dans le régénérateur au niveau de l'appoint de catalyseur, les grains de coke étant de taille comparable à celle du catalyseur de craquage frais. Le problème rencontré pour ce type d'introduction de coke est le problème de la dispersion homogène catalyseur/ coke avant son entrée dans le régénérateur.

Un deuxième objectif de l'invention est d'obtenir que la dispersion du coke au milieu des grains de catalyseur cokés ou sur ceux-ci en cours de régénération soit homogène.

## DESCRIPTION SOMMAIRE DE L'INVENTION

La présente invention s'applique aussi bien à des unités de FCC utilisant un réacteur fonctionnant à courant ascendant (appelé "riser" dans la terminologie anglo-saxonne), qu'à des unités utilisant un réacteur fonctionnant à courant descendant (appelé "downer" dans la terminologie anglo-saxonne).

La présente invention s'applique également à des unités FCC fonctionnant avec un seul réacteur (en écoulement ascendant ou en écoulement descendant), et à des unités FCC fonctionnant avec deux réacteurs ou plus. Généralement, lorsque les unités FCC fonctionnent avec deux réacteurs, un principal et un secondaire, qu'ils fonctionnent en mode maxi essence, maxi GPL ou encore en mode maxi distillat (LCO ou light cycle oil en anglais), ces réacteurs sont à écoulement ascendant, mais une unité qui ferait appel à deux réacteurs à écoulement descendant ou à un réacteur ascendant et un réacteur descendant ne sortirait pas du cadre de la présente invention.

Les caractéristiques techniques essentielles de l'invention sont explicitement définies dans les libellés des revendication indépendantes 1 et 12. D'autres caractéristiques techniques de l'invention sont explicitement définies dans les libellés des revendications dépendantes 2-11 et 13-15.

A cet effet, l'invention concerne un procédé de craquage catalytique d'une charge faiblement cokante de carbone Conradson inférieure ou égale à 0.1 % en poids et de teneur en hydrogène supérieure ou égale à 12,7% en poids, ledit procédé étant mis en œuvre dans une unité comprenant au moins une zone de craquage pour la charge, une zone de séparation/ stripage des effluents des grains de catalyseur cokés et une zone de régénération des dits grains, caractérisé en ce qu'on introduit en amont et/ou au cours de l'étape de régénération du catalyseur au moins un matériau carboné solide à l'état fluidisé, de Carbone Conradson supérieur ou égal à 10% en poids, dans un lit dense de catalyseur coké dans la zone de régénération.

Selon l'invention, le procédé est tel que :

(a) on disperse au moins un matériau carboné solide à l'état fluidisé, de teneur en carbone supérieure ou égale à 80% en poids, sur des grains de catalyseur coké,

(i) en amont de la zone de régénération et en aval de la zone de séparation/ stripage, et/ ou

(ii) dans la zone de régénération du catalyseur au sein des grains de catalyseur coké d'un lit dense,

(b) on ajuste la quantité de matériau carboné solide à l'état fluidisé dispersée au sein des grains de catalyseur coké pour fournir une quantité de coke Qc supplémentaire sur le catalyseur pour satisfaire l'équation (I) suivante :

$$Q_c = Q_t - Q_i \; (I),$$

où Qi est la teneur en coke initiale sur le catalyseur coké après craquage de la charge et où Qt, ou Delta coke, est la teneur en coke nécessaire au maintien de la température du catalyseur régénéré et donc au bilan thermique du procédé,

(c) le mélange de grains de catalyseur coké et de matériau carboné solide est brûlé dans la zone de régénération pour produire un catalyseur régénéré appauvri en matériau carboné,

(d) le catalyseur régénéré est mélangé à la charge faiblement cokante dans la zone de craquage pour produire les grains de catalyseur coké et les effluents,

(e) les grains de catalyseur coké sont séparés des effluents dans la zone de séparation/ stripage puis les grains de catalyseur coké séparés sont envoyés dans la zone de régénération.

Avantageusement, la totalité du matériau carboné ajouté peut être brûlée dans la zone de régénération En effet, si le matériau carboné ajouté n'était pas complètement brûlé, il en résulterait la formation des fines de coke qui se retrouvent en partie dans les cyclones du désengageur de la zone de séparation/strippage et en partie dans les cyclones de la zone de régénération, où ces fines de coke sont responsables d' « afterburning » et de dégradation accélérée des cyclones.

Dans la présente demande, les termes amont et aval se rapportent au sens de circulation envisagé du catalyseur dans le régénérateur.

Le matériau carboné peut être fluidisé par tout moyen, dans un effluent liquide ou gazeux non amalgamant avec le matériau carboné, de préférence de l'air.

Selon l'invention, la quantité de matériau carboné solide à l'état fluidisé dispersée au milieu des grains de catalyseur coké du lit dense est ajustée pour fournir une quantité de coke Qc supplémentaire sur le catalyseur pour satisfaire l'équation (I) suivante :

$$Q_c = Q_t - Q_i \; (I),$$

où Qi est la teneur en coke initiale sur le catalyseur coké après craquage de la charge, et Qt ou Delta coke est la teneur en coke nécessaire au maintien de la température du catalyseur régénéré et donc au bilan thermique du procédé.

En particulier, Qt peut être choisie variant de 0.5 à 1% en poids lorsque la zone de régénération ne comprend qu'une seule étape, et de 0.8 à 1.45% en poids pour une combustion partielle dans le premier étage d'une zone de régénération d'un régénérateur multi-étagé, comprenant au moins deux étapes de régénération.

Le matériau carboné peut être choisi parmi le coke résultant de la cokéfaction du charbon, des cokeurs d'effluents hydrocarbonés de température d'ébullition supérieure à 350°C choisis parmi les coupes lourdes effluents issues de la réaction de craquage principal, HCO (abréviation de « heavy cycle oil ») d'intervalle de distillation compris typiquement entre 360 et 440°C, et slurry d'intervalle de distillation supérieur à 360°C ( noté 360°+), les résidus de biomasse issus de la conversion du bois et/ de la cellulose, le charbon broyé dissout dans un hydrocarbure fluide et/ou injecté par soufflage ou pulvérisation, les coupes riches en asphalte provenant d'unités de désalphatage, les cires non valorisables issues de la liquéfaction du charbon par voie indirecte (GTL « Gas to Liquid ») ou du procédé Fischer Tropsch de transformation du gaz en hydrocarbures ou un mélange des dites coupes.

La charge introduite dans la zone de craquage peut être choisie dans le groupe comprenant les purges d'unité d'hydrocraqueur, ou "bleed", les charges à base coupe gazole de la distillation sous vide de point d'ébullition supérieur à 350°C, et présentant des teneurs en hydrogène supérieures ou égales à 12,7% en poids, les huiles végétales et les hydrocarbures de point d'ébullition inférieur à 160°C, ces charges étant craquées seules ou en mélange dans la zone de craquage du procédé.

Le matériau carboné solide à l'état fluidisé contenant le matériau coké peut être introduit dans la phase dense d'au moins une étape de la zone de régénération lorsqu'elle est multi-étagée.

La dispersion de matériau carboné solide à l'état fluidisé peut être obtenue par des moyens de dispersion de celui-ci sur toute la section de la zone de régénération pour que la distribution des grains de catalyseur sur celle du matériau carboné à l'intérieur de la zone de régénération soit voisine de 1. Autrement dit, la proportion des grains de catalyseur par rapport aux grains de matériau carboné est constante en tout point de la section de la zone de régénération.

A cet effet, la zone de régénération est équipée d'au moins un garnissage structuré, disposé en amont et/ou en aval de moyens de dispersion du matériau carboné par rapport à la circulation envisagée du catalyseur dans ladite zone de régénération, et ce quels que soient les moyens de dispersion, ledit garnissage structuré est formé tel que défini dans les libellés des revendication indépendantes 1 et 12. Avantageusement, ce garnissage structuré sera situé en amont des moyens de dispersion du matériau carboné.

La zone de régénération peut être équipée d'au moins un garnissage structuré pour la dispersion homogène des grains de catalyseur coké et la dispersion de matériau carboné solide à l'état fluidisé peut être effectuée à contrecourant de la circulation de catalyseur en aval du dit garnissage structuré, celui-ci couvrant tout ou partie de la section de ladite zone de régénération.

La dispersion homogène peut par exemple être réalisée en présence d'au moins un garnissage placé dans la phase dense d'un premier étage de la zone de régénération dans le cas d'une zone de régénération multi-étagée.

Le matériau carboné solide à l'état fluidisé peut être dispersé sur tout ou partie de la hauteur de chaque lit dense de la zone de régénération, chaque dispersion se faisant après homogénéisation du lit fluidisé, ce dernier étant équipé éventuellement d'au moins un garnissage structuré.

L'invention concerne également un dispositif de mise en œuvre du procédé selon l'invention comprenant au moins un réacteur principal et éventuellement au moins un réacteur secondaire, au moins un désengageur/stripeur, un régénérateur mono ou multi-étagé, caractérisé en ce qu'il comprend des moyens de dispersion homogène d'un matériau carboné à l'état divisé sur des grains de catalyseur coké issus du désengageur/strippeur, ces moyens de dispersion homogène étant situés en amont du régénérateur, et/ou dans le régénérateur lui-même. Selon l'invention, le régénérateur est en outre équipé d'au moins un garnissage structuré, disposé en amont et/ou en aval des moyens de dispersion du matériau carboné par rapport à la circulation envisagée du catalyseur dans ledit régénérateur

Les moyens de dispersion du matériau carboné peuvent être placés sur une conduite reliant le stripeur au régénérateur et acheminant le catalyseur coké et strippé au dit régénérateur

Les moyens de dispersion du matériau carboné peuvent encore être placés au niveau d'une partie dense du lit fluidisé dans le régénérateur.

Eventuellement, des moyens de dispersion du matériau carboné peuvent être placés aux deux emplacements décrits précédemment.

Lorsque les moyens de dispersion sont placés sur une conduite reliant le stripeur au régénérateur et acheminant le catalyseur coké et strippé au dit régénérateur, ils peuvent comprendre une trémie de stockage de matériau carboné préalablement broyé à la granulométrie désirée. Il est notamment possible d'utiliser la même trémie pour stocker et injecter à la fois le catalyseur qu'il soit frais ou d'équilibre (issu de la zone de craquage) et les particules de matériau carboné.

Lorsque les moyens de dispersion sont placés dans le régénérateur, ils peuvent être choisis parmi les moyens susceptibles de disperser les mélanges gaz/solides dans un lit dense. Il s'agit de préférence de tubes ouverts et/ou de râteaux formés de plusieurs tubes parallèles s'ouvrant dans le lit dense, ces tubes étant reliés à un tube distributeur.

Le régénérateur pourra être équipé d'au moins un garnissage structuré, disposé en amont et/ou en aval de moyens de dispersion du matériau carboné par rapport à la circulation envisagée du catalyseur dans le dit régénérateur.

En particulier, le régénérateur peut être équipé d'au moins un garnissage structuré, disposé en amont des moyens de dispersion du matériau carboné par rapport à la circulation envisagée du catalyseur dans le dit régénérateur, au niveau du lit fluidisé dense du premier étage de régénération.

Ces garnissages sont formés par un enchevêtrement de plaques, de ruban ou d'ailettes constituant un tamis, ce tamis occupant moins de 10% en surface de la section de la capacité dans laquelle il est placé, mais recouvrant en projection sur celle-ci toute sa surface.

## DESCRIPTION DETAILLEE DE L'INVENTION

Les charges que peut traiter une unité de FCC selon la présente invention sont des charges à carbone Conradson inférieur ou égal à 0,1 % en poids et ayant une teneur en hydrogène supérieure ou égale à 12,7 % en poids.

La présente invention peut se décrire comme un procédé de craquage catalytique d'une charge faiblement cokante de carbone Conradson inférieur ou égal à 0.1% en poids et de teneur en hydrogène supérieure ou égale à 12,7% en poids, ce procédé étant mis en œuvre dans une unité comprenant au moins une zone de craquage pour la charge, une zone de séparation/stripage des effluents des grains de catalyseur cokés et une zone de régénération des dits grains, la caractéristique du procédé étant qu'on introduit en amont et/ou au cours de l'étape de régénération du catalyseur au moins un matériau carboné solide sous forme à l'état fluidisé, dont la teneur en carbone est supérieure ou égale à 80% en poids, dans un lit fluidisé dense de grains de catalyseur cokés.

Selon l'invention, le procédé est tel que :

(a) on disperse au moins un matériau carboné solide à l'état fluidisé, de teneur en carbone supérieure ou égale à 80% en poids, sur des grains de catalyseur coké,

(i) en amont de la zone de régénération et en aval de la zone de séparation/ stripage, et/ ou
(ii) dans la zone de régénération du catalyseur au sein des grains de catalyseur coké d'un lit dense,

(b) on ajuste la quantité de matériau carboné solide à l'état fluidisé dispersée au sein des grains de catalyseur coké pour fournir une quantité de coke Qc supplémentaire sur le catalyseur pour satisfaire l'équation (I) suivante :

$$Q_c = Q_t - Q_i \text{ (I)},$$

où Qi est la teneur en coke initiale sur le catalyseur coké après craquage de la charge et où Qt, ou Delta coke, est la teneur en coke nécessaire au maintien de la température du catalyseur régénéré et donc au bilan thermique du procédé,

(c) le mélange de grains de catalyseur coké et de matériau carboné solide est brûlé dans la zone de régénération pour produire un catalyseur régénéré appauvri en matériau carboné,

(d) le catalyseur régénéré est mélangé à la charge faiblement cokante dans la zone de craquage pour produire les grains de catalyseur coké et les effluents,

(e) les grains de catalyseur coké sont séparés des effluents dans la zone de séparation/ stripage puis les grains de catalyseur coké séparés sont envoyés dans la zone de régénération.

Pour diviser, voire fluidiser, les particules de matériau carboné solide dans un effluent liquide ou gazeux, on utilise tout moyen comparable à ce qui est utilisé notamment pour la fluidisation de catalyseur, le dit effluent devant maintenir divisés et non amalgamés les grains du dit matériau. De préférence, on utilisera l'air comme effluent de fluidisation de ce matériau carboné solide à l'état divisé.

Par zone de régénération, on entend une zone dans laquelle on réalise une régénération du catalyseur coké en une ou plusieurs étapes, généralement deux, dans une même capacité comprenant un ou plusieurs étages, et/ou dans des capacités différentes de régénération comprenant une ou plusieurs étapes dans un ou plusieurs étages.

La zone de craquage, dédiée au craquage de charge, comprend au moins un réacteur, notamment au moins un réacteur principal et au moins un réacteur secondaire.

La zone de séparation/strippage est dédiée à la séparation et au strippage des grains de catalyseur coké et des effluents issus du craquage de la charge. Cette zone de séparation/strippage comprend au moins un désengageur et au moins un stripeur.

Une charge faiblement cokante est une charge qui produira un catalyseur faiblement coké en sortie du réacteur de craquage dont la quantité de coke n'est pas suffisamment importante pour assurer le bilan thermique de l'unité de craquage catalytique dans lequel il est utilisé, typiquement inférieure à 0.4% poids. En effet, la régénération du catalyseur par combustion du coke dégage de la chaleur qui doit être récupérée en quantité suffisante par le catalyseur pour que ce dernier apporte d'une part l'énergie suffisante à la vaporisation quasi complète de la charge injectée sous forme liquide à l'entrée du réacteur et apporte d'autre part l'énergie suffisante aux réactions de craquage globalement endothermiques afin d'assurer une température de réaction en sortie dudit réacteur généralement comprise entre 480 et 650°C selon les configurations et les objectifs de conversion recherchés.

Pour permettre la dispersion de ce matériau carboné, la présente invention utilisera tous moyens permettant la dispersion homogène de matériau solide comme ceux actuellement utilisés pour l'introduction de catalyseur frais dans un lit dense de catalyseur. Pour une meilleure compatibilité entre grains de catalyseur coké et grains du matériau coké, on visera une taille des grains pour le matériau carboné le mieux adaptée pour permettre à la fois d'obtenir un bon comportement de fluidisation mais également de limiter l'élutriation des particules de matériau fortement carboné dans la phase diluée au dessus du lit dense. A cet effet, le ou les matériaux carbonés ajoutés peuvent avantageusement présenter une taille de particules sensiblement identique à la taille des grains de catalyseur. Par sensiblement identique, on entend une taille identique avec une variation de ±10%.

L'avantage de la présente invention est essentiellement d'augmenter la quantité de coke dans le régénérateur permettant ainsi de compenser le peu de coke formé par la charge au sein du réacteur de craquage. Cette augmentation du coke à brûler dans le régénérateur, de teneur en hydrogène très faible, a pour effet d'augmenter la chaleur résultant de la combustion du coke et conséquemment d'augmenter la température des grains de catalyseur ainsi régénérés qui vont être recyclés au réacteur principal dans une configuration à un seul réacteur et aux réacteurs principal et secondaire dans une configuration à deux réacteurs. L'avantage ultime est de permettre d'ajuster à la demande la quantité de coke nécessaire à l'équilibre thermique de l'unité et assurer ainsi son bon fonctionnement.

Pour un bon fonctionnement de l'unité de FCC alimentée avec une charge peu cokante, la quantité de coke (Qt) présent sur le catalyseur entrant dans la zone de régénération, nécessaire à l'équilibrage du bilan thermique va correspondre à la somme de la quantité initiale de coke amenée par le craquage de la charge (Qi) sur le catalyseur (dans le réacteur principal ou dans les réacteurs de l'unité FCC, au nombre de deux ou plus) et de la quantité de coke apportée par fluidisation du matériau carboné ($Q_c$) sur le catalyseur coké après craquage de la charge. Généralement, Qt, la quantité de coke entrant dans le régénérateur typique pour un bilan thermique équilibré est maintenue entre 0.5 et 1 % en poids lorsque la combustion est dans le cas d'une zone de régénération à une seule étape, et entre 0.8 et 1.45 % en poids pour une combustion partielle dans la première étape d'une zone de régénération d'un régénérateur multi-étagé, comprenant au moins deux étapes de régénération.

Dans le cadre de la présente invention, la quantité de matériau carboné dispersée au milieu des grains de catalyseur cokés est ajustée pour fournir une quantité de coke Qc supplémentaire sur le catalyseur pour satisfaire l'équation (I) suivante :

$$Q_c = Q_t - Q_i \text{ (I)},$$

où Qi est la teneur en coke initiale sur le catalyseur coké après craquage de la charge, et Qt, ou Delta coke, est la teneur en coke nécessaire au maintien de la température du catalyseur régénéré et donc au bilan thermique du procédé.

Pour mettre en œuvre l'invention, le matériau carboné dont la teneur en carbone est supérieure ou égale à 80 % en poids, peut être choisi parmi :

- du coke de teneur en hydrogène inférieure ou égale à 10% en poids, ce coke étant issu de la cokéfaction du charbon, des cokeurs d'effluents hydrocarbonés de température d'ébullition supérieure à 350°C choisis parmi les coupes lourdes effluents issues de la réaction d'une réaction de craquage, HCO (abréviation de heavy cycle oil) d'intervalle de distillation compris typiquement entre 360 et 440°C, et slurry d'intervalle de distillation supérieur à 360°C ( noté 360°+),
- des résidus de biomasse issus de la conversion du bois et/ de la cellulose,
- le charbon broyé dissout dans un hydrocarbure fluide et/ou injecté par soufflage ou pulvérisation,
- les coupes riches en asphalte provenant d'unités de désalphatage,
- les cires non valorisables issues de la liquéfaction du charbon par voie indirecte (GTL) ou du procédé Fischer Tropsch de transformation du gaz en hydrocarbures ou un mélange des dites coupes.

Parmi les charges faiblement cokantes qu'il est possible de traiter selon la présente invention, on peut trouver :

- les purges d'unité d'hydrocraqueur, dénommées "bleed" dans la terminologie anglo-saxonne, présentant une teneur en hydrogène supérieure ou égale à 12,7% en poids,
- les charges VGO (abréviation de gasoil sous vide résultant de la distillation sous vide des résidus de distillation atmosphérique) prétraitées sévèrement, ayant généralement un point d'ébullition supérieur à 350°C, et présentant des teneurs en hydrogène supérieures ou égales à 12,7% en poids,
- les huiles végétales,
- des hydrocarbures de point de distillation inférieur ou égal à 160°C comme les essences, voire certaines molécules de gaz liquéfié telles le butane, provenant d'unités de distillation et ou de conversion.

Ces charges peuvent être craquées seules ou en mélange dans le réacteur principal de l'unité de craquage catalytique.

Dans la présente invention, il s'agit de produire des effluents comme par exemple l'essence et des GPL (gaz de pétrole liquéfié) à partir d'une charge peu cokante, telle qu'une de celles citées précédemment, par craquage catalytique (FCC) dans une unité correspondante qui possède au moins un réacteur principal fonctionnant à courant ascendant (appelé "riser") ou à courant descendant (appelé "downer"), le catalyseur coké en sortie du réacteur étant introduit dans une zone de séparation/ stripage à la sortie de laquelle le catalyseur coké est récupéré et envoyé dans le régénérateur de l'unité. Le régénérateur peut être mono ou multi étagé. Dans le cas d'un réacteur mono-étagé, ce dernier comprend au moins un lit fluidisé de grains de catalyseur cokés dans lequel a lieu la combustion du coke, se répartissant en fonction de leur densité moyenne respective dont au moins un lit en phase dense dans lequel se produit l'essentiel de la combustion, et un lit en phase diluée où les grains de catalyseurs en tout ou partie décokés se séparent des effluents gazeux résultant de la combustion. C'est dans la partie du lit fluidisé dense dans laquelle la réaction de combustion est la plus complète que le catalyseur peut atteindre la température visée avant son recycle au réacteur principal. C'est donc au niveau du lit dense que le matériau carboné est introduit sous forme fluidisée.

Pour que le matériau carboné introduit sous forme fluidisée se mélange de façon homogène au milieu des grains de catalyseur, il est nécessaire de disperser les grains de matériau carboné sur toute la section du lit par des moyens propres à cette dispersion, de façon à ce que la distribution des grains de catalyseur sur celle des particules de matériau carboné soit voisine de 1. Un des moyens permettant d'arriver à un tel rapport est de rendre homogène la phase dense du lit fluidisé par insertion de garnissages structurés améliorant la dispersion des grains de catalyseurs cokés en amont de la dispersion des grains de matériau carboné, cette dispersion étant obtenue par tous moyens. Ces garnissages structurés peuvent couvrir tout ou partie de la section du régénérateur et sur au moins une partie de la hauteur de celle-ci. On pourrait ainsi envisager d'introduire de façon étagée ou non, du matériau carboné sur tout ou partie de la hauteur du lit dense, chaque dispersion de matériau carbonée se faisant après homogénéisation du lit fluidisé au moyen d'un garnissage structuré.

L'emploi des garnissages structurés permet d'assurer un flux de catalyseur continu de densité homogène. D'accord avec les caractéristiques techniques essentielles de l'invention, les garnissages occupent moins de 10% de la surface de la section de passage dans la capacité dans laquelle ils sont placés, bien qu'en projection sur celle-ci ils en occupent toute la surface.

Un des avantages liés à l'utilisation d'un tel garnissage est de faciliter l'homogénéisation et la combustion du matériau carboné injecté dans la phase dense, ce qui permet de limiter l'apparition de points chauds dans le lit fluidisé. Un autre avantage est de limiter l'entraînement de particules carbonées incomplètement brûlées dans les gaz en sortie du régénérateur.

Lorsqu'on opère avec un régénérateur multi-étagé comprenant au moins deux étages de régénération, le premier étage vise la combustion partielle du coke présent sur les grains de catalyseur issus du réacteur, cette combustion partielle conduisant à la formation de CO et surtout de vapeur d'eau à partir des hydrogènes présents. Comme la combustion est partielle même s'il y a formation de vapeur d'eau à proximité des grains de catalyseurs, la température de réaction est moins élevée et la probabilité de fragiliser l'activité des grains de catalyseur est moins grande. L'ajout de matériau carboné de teneur en hydrogène inférieure à 10% en poids, ne perturbera pas la combustion partielle si la quantité de matériau carboné par rapport à celle des grains cokés de catalyseur est équivalente, soit dans un rapport massique voisin de 1. Le second étage se comporte quant à lui comme un régénérateur mono étagé en combustion complète.

Dans le cadre de la présente invention, il est possible d'injecter à l'état fluidisé du matériau carboné aussi bien dans le premier étage que dans le second étage de régénération mais toujours dans la phase dense du lit fluidisé contenu dans ces étages. Cette injection est améliorée par l'introduction de garnissages structurés tels que décrits précédemment en amont de la dispersion du matériau carboné dans le dit lit. On préférera d'ailleurs l'injection de matériau carboné au premier étage du régénérateur pour disposer de plus de temps (celui des deux étages de régénération) pour finaliser la combustion des particules de matériau carboné.

Cependant, l'injection de coke provenant notamment de cokeurs, pose d'autres problèmes de fonctionnement de l'unité. Le coke comme le coke ex-cokeur, encore appelé « petcoke », contient généralement beaucoup de métaux lourds de type Nickel et Vanadium qui sont des poisons pour l'activité catalytique du catalyseur utilisé dans les unités de FCC, mais aussi du soufre et de l'azote dont on doit limiter la quantité. On sait que la quantité de métaux lourds tolérable dans le catalyseur d'une unité de FCC est, pour un régénérateur à deux étages, d'au plus 10000ppm, de préférence de moins de 6500ppm, et, pour un régénérateur mono étagé, de 5000 à 7000ppm. Pour contrôler la quantité de métaux lourds accumulés dans le catalyseur, on injecte un complément de catalyseur frais ou d'équilibre, appelé « flush cat » par l'homme du métier, dans le régénérateur de façon à diminuer la quantité de métaux circulant dans l'unité. La présence d'azote et de soufre dans le coke injecté au régénérateur conduira inévitablement à la formation d'espèces polluantes non désirées dans les fumées sortant du régénérateur, telles que NOx et SOx. Pour en limiter la formation lors de la combustion du coke, on pourra recourir à l'utilisation d'additifs commerciaux dits DeSOx connus par l'homme du métier. Ces additifs, commercialisés par tous les vendeurs de catalyseurs, permettent de capter le $SO_2$ dans le régénérateur et le transporter vers la zone réactionnelle où, en présence de vapeur d'eau, le $SO_2$ est alors libéré sous forme d'acide sulfureux, $H_2S$, qui sera alors récupéré dans la section de lavage des gaz craqués. Pour les NOx, il sera préféré une solution type SCR (Selective Catalytic Réduction ou réduction catalytique sélective) ou SNCR (Selective Non Catalytic Réduction ou réduction catalytique non-sélective). Enfin, citons les unités de lavage des fumées de type scrubber qui peuvent abattre à la fois les NOx et les SOx ainsi que les particules de catalyseur. Dans tous les cas, il sera nécessaire de faire une étude technico-économique de manière à sélectionner l'option de traitement de fumées la plus adaptée en fonction de la législation en matière de rejets.

La présente invention a également pour objet un dispositif de mise en œuvre de l'invention comprenant les différentes capacités nécessaires à la mise en œuvre d'un procédé de craquage catalytique, c'est-à-dire, au moins un réacteur principal et éventuellement au moins un réacteur secondaire, au moins un désengageur et un stripeur, un régénérateur mono ou multi-étagé, le dit dispositif comprenant des moyens de dispersion homogènes de matériau carboné à l'état divisé sur les grains de catalyseur cokés dans le régénérateur lui-même. lui-même dont les caractéristique techniques essentielles sont définies dans le libellé de la revendication indépendante 12. Ces moyens de dispersion homogène sont par exemple ceux utilisés pour l'introduction de catalyseur frais dans un lit dense de catalyseur.

Ces moyens de dispersion peuvent comprendre une trémie de stockage de matériau carboné préalablement broyé à la granulométrie désirée. Les particules de matériau carboné seront préférentiellement maintenues à l'état fluidisé par injection d'air dans la trémie. De cette trémie, un système d'injection permet de transporter les particules de matériau carboné vers le régénérateur à l'aide d'un gaz de transport, typiquement de l'air, dont le débit est ajusté par un orifice de restriction et qui permet de contrôler la quantité de matériau carboné envoyée vers la phase dense du régénérateur. Il s'agit ici d'un système analogue à celui utilisé pour introduire le catalyseur frais dans toute unité de FCC et connu par l'homme du métier. Citons, dans une des variantes possibles de l'invention, qu'il est possible d'utiliser la même trémie pour stocker et injecter à la fois le catalyseur qu'il soit frais ou d'équilibre et les particules de matériau carboné.

Les moyens de dispersion, qui sont placés dans le régénérateur, peuvent également être choisis parmi les moyens susceptibles de disperser les mélanges gaz/ solides dans un lit dense. Il s'agit de préférence de tubes ouverts et/ou de râteaux formés de plusieurs tubes parallèles s'ouvrant dans le lit dense, ces tubes étant reliés à un tube distributeur.

Selon l'invention, le régénérateur est en outre équipé d'au moins un garnissage structuré, disposé en amont et/ou en aval des moyens de dispersion du matériau carboné par rapport à la circulation envisagée du catalyseur dans ledit régénérateur.

Dans un premier mode de réalisation du dispositif, les moyens de dispersion du matériau carboné sont placés sur une conduite reliant le stripeur au régénérateur et acheminant le catalyseur coké et strippé au dit régénérateur. On peut disposer avantageusement au moins un garnissage structuré dans la dite conduite entre stripeur et régénérateur, après le point d'injection des particules de matériau carboné (par rapport à la circulation envisagée du catalyseur dans le dit régénérateur), pour assurer une meilleure homogénéisation du mélange matériau carboné/ catalyseur.

Dans un deuxième mode de réalisation du dispositif, les moyens de dispersion sont placés avantageusement au niveau de la partie du lit dense. Le régénérateur sera équipé d'au moins un garnissage structuré, disposé en aval et/ou en amont des moyens de dispersion du matériau carboné par rapport à la circulation envisagée du catalyseur dans le dit régénérateur.

Dans une variante, plusieurs garnissages, chacun étant associé à un moyen de dispersion de matériau carboné, peuvent se succéder avec au moins un garnissage associé à un moyen de dispersion.

Comme élément de garnissage en accord avec les caractéristiques techniques essentielles dans les libellés des revendications indépendantes 1 et 12, on pourrait utiliser un ou plusieurs des garnissages structurés décrits dans les brevets EP719850, US7022221, US7077997, WO2007/09477l, WO00/35575 et CN1763150. Il s'agit ici dans chacun des garnissages envisagés d'aérer le flux de grains cokés en leur faisant suivre des chemins privilégiés obtenus par un enchevêtrement de plaques, de ruban ou d'ailettes constituant un tamis. La section de ce tamis parallèle à la section de la capacité qui le contient, occupe moins de 10% de la surface de la section de passage de la dite capacité, et recouvre en projection sur celle-ci toute la surface de sa section. Ces enchevêtrements sont disposés généralement en couches de même type, permettant de contrôler cet aérage des grains.

En amont des moyens de dispersion du matériau carboné, le matériau carboné brut est broyé finement, puis tamisé et seuls les grains présentant la taille requise, soit approximativement la taille des grains de catalyseur frais, sont envoyés dans un conduit dans lequel est injecté un gaz de transport, typiquement de l'air, qui va entraîner le solide divisé vers les dispositifs de dispersion dans le catalyseur coké.

Le dimensionnement d'un tel dispositif de dispersion suit les mêmes règles que celles utilisées par l'homme du métier pour assurer le transport pneumatique d'un solide finement divisé, comme le catalyseur, de sa trémie de stockage vers son lieu d'injection dans un lit dense. Parmi les moyens susceptibles de disperser les mélanges gaz/solides dans un lit dense, on utilisera de préférence des tubes ouverts et/ou des râteaux formés de plusieurs tubes parallèles s'ouvrant dans le lit dense, ces tubes étant reliés à un tube distributeur.

Avec le dispositif de dispersion du matériau carboné dans le lit dense, le régénérateur pourra être opéré en combustion totale ou partielle, en présence d'un gaz contenant de l'oxygène. Pour une marche en combustion partielle, l'air injecté ne pourra pas brûler l'intégralité du coke présent dans le régénérateur, provenant à la fois du coke sur les grains de catalyseur coké et des particules de matériau carboné injectées volontairement dans le régénérateur. Dans ce cas, des particules de coke iront vers la zone réactionnelle. L'avantage d'une telle situation est de pouvoir diluer l'activité catalytique de la masse de catalyseur circulant et donc de réduire le niveau de conversion de la charge traitée pour maximiser la production de distillât. Un autre avantage de cette marche en combustion partielle est que le bilan thermique n'est pas significativement modifié puisque les particules de coke sont à la température de la phase dense dans le régénérateur.

L'invention est maintenant décrite en référence aux dessins annexés, non limitatifs, dans lesquels :

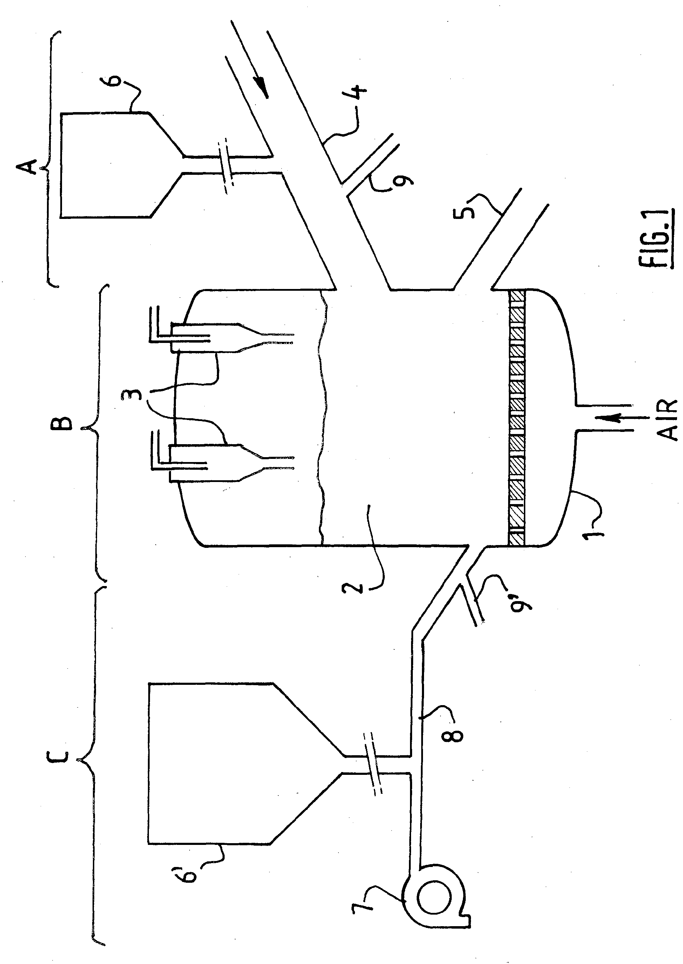

- la figure 1 est une coupe d'un régénérateur équipé d'un système de dispersion de grains de matériau carboné dans un fluide gazeux jusqu'à l'entrée du dispositif de dispersion dans celui-ci : deux types de montage sont possibles AB et BC selon que l'injection de matériau carboné est faite dans la conduite entre le stripeur et le régénérateur ou directement dans le régénérateur (par exemple par une autre conduite).

La figure 1 comporte dans sa partie principale B un régénérateur (1) contenant un lit dense de catalyseur (2) équipé de deux cyclones (3) pour une dernière séparation gaz solide avant l'évacuation du gaz de combustion chargé en $CO_2$. Le régénérateur (1) est équipé d'une arrivée de catalyseur coké (4), d'une conduite de sortie du catalyseur régénéré (5) et d'une arrivée d'air en fond. Le régénérateur (1) est couplé avec un système d'introduction de matériau carboné, par exemple du coke, selon deux types de configuration possible AB ou BC. Ces deux systèmes d'introduction du matériau carboné correspondant aux parties A et C sont représentés sur la figure 1. Dans chaque partie A ou C, le matériau carboné est broyé dans des capacités (6 ou 6') puis, le matériau carboné sous forme de particules pulvérisées arrive dans une conduite (4) pour la mise en œuvre AB, ou une conduite (8) dans la configuration BC. Dans cette dernière configuration, la conduite (8) est équipée d'un souffleur pneumatique (7) susceptible de maintenir les particules de matériau carboné introduites à l'état fluidisé circulant jusqu'au régénérateur où le matériau carboné est mélangé avec le lit dense de catalyseur coké. Les conduites (4) et (8) sont équipées d'injecteurs (9) et (9') pour l'introduction d'additifs DeSox et DeNOx.

Des exemples sont donnés ci-après en vue d'illustrer l'invention mais ils ne peuvent être interprétés comme des limitations de l'invention.

EXEMPLE

Le présent exemple montre les avantages de la présente invention en comparant l'efficacité en rendement de produits lorsqu'on craque des charges faiblement cokantes dans une unité de FCC avec ou sans recycle de coupes cokantes.

La production de coke au coker est de 250 t/h de composition suivante: C=85.2%, H=3.6% (en poids), N=1% (en poids), S=7.5% (en poids), Ni=179ppm (poids) et V=565ppm (poids). Le pouvoir calorifique du coke est supposé égal à 7.75 kcal/kg. C'est ce coke qui est utilisé dans le présent exemple.

On distingue un cas de base sans injection de coke avec une unité de craquage catalytique (FCC) à un seul réacteur ascendant ou "riser" d'une capacité de 4800 tonnes par jour, soit 200 tonnes par heure, et traitant une charge correspondant du VGO hydrotraité dont les propriétés sont données ci-après.

Tableau 1 : propriétés du VGO hydrotraité

| Charge | | VGO hydrotraité |
|---|---|---|
| Densité | $g/cm^3$ | 0,8610 |
| Teneur en H2 | %poids | 13.7 |
| Soufre | ppm poids | 330 |
| Azote | ppm poids | 550 |
| CCR (Carbone Conradson) | % poids | <0,1 |
| Ni | ppm poids | <2 |
| V | ppm poids | <2 |

Des essais sur pilote ont montré que cette charge produisait très peu de coke, environ 3.3% pour une température de réaction de 525°C et un C/O de 8. Sur la base de ces données pilote, nous avons réalisé des calculs de bilan thermique dans différents cas de fonctionnement d'une unité industrielle, qui, par définition, doit boucler son bilan thermique. Les résultats de ces calculs sont consignés dans le tableau 2 suivant. Les calculs de bilan thermique ont été effectués sur la base des formules de calculs mentionnées dans le recueil : »Fluid catalytic cracking handbook », seconde édition de 2000, par Reza SADEGHBIGI, publié par Gulf Professional publishing.

Tableau 2

| | | Cas 1 | Cas 2 | Cas 3 |
|---|---|---|---|---|
| Débit de charge | t/h | 200,0 | 200,0 | 200,0 |
| Eq. Ni | ppm | 0,1 | 4,0 | 2,9 |
| Eq.V | ppm | 0,1 | 12,7 | 9,1 |
| Taux d'addition de catalyseur frais | t/jour | 2,00 | **10,60** | **8,20** |
| Surface active du catalyseur | $m^2/g$ | 147,3 | 146,5 | 146,9 |

(suite)

| | | Cas 1 | Cas 2 | Cas 3 |
|---|---|---|---|---|
| Teneur en Ni sur catalyseur | ppm | 120 | 1811 | 1698 |
| Teneur en V sur catalyseur | ppm | 120 | 5660 | 5327 |
| Température de réaction (ROT) | °C | 525,0 | 525,0 | 525,0 |
| Débit de catalyseur Ratio C/O % coke sur catalyseur (delta coke) | t/min % poids | 26,8 8,03 0,41 | 24,3 7,29 0,45 | 24,4 7,32 0,45 |
| Température préchauffe charge | °C | **416,4** | **208,2** | **273,7** |
| Delta Température entrée sortie four | delta °C | 208,2 | 0,0 | 65,5 |
| Energie fournie par four de charge | Mkcal/h | 32,4 | 0,0 | 9,3 |
| température phase Dense (=TRG) | °C | **627,2** | **714,4** | **691,6** |
| **Energie à fournir au régénérateur** | **Mkcal/h** | **0** | **35** | **25** |
| | | | | |
| Std CONVERSION | % poids | 83,8 | 83,9 | 83,7 |
| H2S | % poids | 0,01 | 0,01 | 0,01 |
| H2 | % poids | 0,00 | 0,01 | 0,01 |
| C1-C2 | % poids | 1,40 | 1,45 | 1,45 |
| C3-C4 | % poids | 23,4 | 23,4 | 23,3 |
| Standard LCN C5-160 | % poids | 44,1 | 44,1 | 44,0 |
| Standard HCN 160-220 | % poids | 11,7 | 11,7 | 11,7 |
| Standard LCO 220-360 | % poids | 11,5 | 11,5 | 11,6 |
| Standard Slurry 360+ | % poids | 4,7 | 4,7 | 4,7 |
| Coke | % poids | 3,3 | 3,3 | 3,3 |
| Total | % poids | 100,0 | 100,0 | 100,0 |
| | | | | |
| **Coke injecté au régénérateur** | **kg/h** | **0** | **4502** | **3215** |
| Rendement Coke équivalent | % poids | 3,3 | 5,5 | 4,9 |
| Débit d'air injecté au régénérateur | t/h | **91** | **152** | **135** |

Dans le tableau 2 sont consignés trois cas de fonctionnement d'une unité industrielle.

Dans la première colonne intitulée « cas 1 ou cas de base sans injection de coke », on n'injecte pas de coke au régénérateur et on a calculé la température de préchauffe de la charge injectée au réacteur nécessaire pour obtenir 3.3% (poids) de coke avec une température de réaction de 525°C. Dans ce cas, pour obtenir le bilan thermique de l'unité, la température de préchauffe de la charge doit être très élevée et par ailleurs inacceptable, car au-delà de 400°C, la charge commence à craquer avant même son entrée dans le réacteur de l'unité. De plus, la température de la phase dense du régénérateur serait à peine de 627°C, température également inacceptable car inférieure à la température d'initiation de la combustion du coke déposé sur le catalyseur contenu dans le régénérateur.

Deux autres cas ont été envisagés pour permettre d'atteindre à la fois des températures de préchauffe de la charge injectée dans le réacteur et du catalyseur coké dans la phase dense dans le régénérateur acceptables avec un fonctionnement d'une unité de FCC industrielle, présentant un bilan thermique équilibré.

Dans la configuration du cas 2 du tableau 2, il y a injection de coke dans le régénérateur sans four annexe de préchauffe de la charge, celle-ci étant préchauffée uniquement par une série d'échangeurs charge/effluent. Dans ce cas, la température de préchauffe ne peut excéder 208°C. Par conséquent, pour obtenir une température du catalyseur coké dans la phase dense du régénérateur suffisamment élevée, typiquement supérieure à 650°C, et atteindre un bilan thermique équilibré dans l'unité, il est nécessaire de fournir de l'énergie par combustion de coke supplémentaire. Dans ce cas, un apport de 35 Mkcal/h au régénérateur permet alors d'obtenir une température de phase dense de 714°C. Pour un tel apport de calories, il faudra alors injecter environ 4500 kg/h de coke ex coker au sein du catalyseur coké à régénérer.

L'inconvénient de l'injection de coke ex coker au régénérateur est l'introduction de métaux tels que le Ni et le V, connus pour être des poisons du catalyseur ayant pour effet de désactiver le catalyseur. Connaissant le débit d'injection de coke ex coker au régénérateur et les teneurs en Ni et V, il est alors possible de calculer la teneur en Ni et V équivalente rapportée à la charge déposée sur le catalyseur en recirculation. Cet exercice nous permet de calculer l'appoint de catalyseur qu'il faudra réaliser pour conserver un niveau d'activité catalytique satisfaisant à l'intérieur de l'unité de craquage. Pour pouvoir se comparer avec le cas 1, l'appoint de catalyseur est ajusté jusqu'à obtenir le même niveau de surface active, soit environ 147 m$^2$/g. On peut voir dès lors que comparativement au cas de base, il faudra augmenter l'appoint de catalyseur de 2 à 10.6 t/jour, représentant un coût supplémentaire de mise en œuvre de l'invention non négligeable.

Pour limiter ce surcoût de fonctionnement lié à l'introduction d'un appoint plus élevé de catalyseur, On peut réduire l'énergie apportée au régénérateur par l'introduction de coke ex coker en augmentant celle apportée par la charge au réacteur. C'est le cas 3 qui consiste à injecter du coke au régénérateur tout en préchauffant la charge avec un four de préchauffe présent sur la conduite d'arrivée de la charge en amont du réacteur de craquage. Si l'énergie apportée au régénérateur passe à 25 Mkcal/h comparativement à 35 Mkcal/h, cela permettra de réduire la quantité de coke ex coker à injecter au régénérateur jusqu'à 3200 kg/h. En faisant le même exercice que précédemment, l'appoint de catalyseur frais sera alors réduit à 8.2 t/j comparativement à 10.6 t/jour. Si l'énergie au régénérateur est ainsi réduite pour un rendement coke produit par la charge équivalent, pour atteindre un bilan thermique acceptable de l'unité, il est alors nécessaire d'apporter de l'énergie au catalyseur au niveau du réacteur de craquage en préchauffant davantage la charge. Dans ce cas, la température de préchauffe doit être d'environ 274°C. Si la température de préchauffe maximum de la charge en sortie des échangeurs charge/effluent est de 208°C, on ajoutera, après les échangeurs, un four sur la conduite d'arrivée de la charge à craquer pour augmenter la température de celle-ci de 208°C jusqu'à 274°C, ce qui nécessitera 9.3 Mkcal/h d'apport de chaleur à la charge. Dans ce cas, le calcul du bilan thermique montre qu'on atteint ainsi l'équilibre thermique de l'unité puisque la baisse d'énergie apportée par l'ajout de coke au régénérateur d'environ 10 Mkcal/h, est compensée par l'apport en énergie via la préchauffe de la charge, par l'intermédiaire d'un four.

Les gains de coût de fonctionnement liés à la diminution de l'appoint de catalyseur sont alors à pondérer par l'augmentation des coûts de fonctionnement liés à l'utilisation de combustible de chauffage au niveau du four pour chauffer la charge. Sur un plan économique, le cas 3 n'est pas nécessairement meilleur que le cas 2. En effet, la somme des coûts pour l'investissement lié à l'installation d'un four, et pour la consommation d'un combustible de meilleure qualité brûlé dans ledit four supplémentaire est au moins égale sinon supérieure au coût de l'ajout de coke ex coker broyé comme décrit dans le cas 2 avec un appoint de catalyseur frais augmenté.

Dans ce cas 3, la température au régénérateur est à peine supérieure à 690°C : il sera difficile de baisser davantage le volume de coke injecté au régénérateur donc l'énergie apportée par celui-ci sans risquer de compromettre le bon fonctionnement du régénérateur, c'est-à-dire une combustion complète du coke présent sur le catalyseur à régénérer.

On notera également que les rendements en produits de craquage restent équivalents dans les trois cas envisagés, si ce n'est une légère augmentation du volume de gaz secs pour les cas 2 et 3 avec injection de coke, cet accroissement étant lié à la présence de métaux sur le catalyseur.

Enfin, en calculant le rendement coke équivalent rapporté à la charge, on peut estimer le débit d'air nécessaire au régénérateur pour permettre la combustion simultanée du coke déposé sur le catalyseur suite au craquage de la charge dans le réacteur et le coke ex coker ajouté au régénérateur, et cela à iso-excès d'oxygène dans les fumées.

**Revendications**

1. Procédé de craquage catalytique d'une charge faiblement cokante de carbone Conradson inférieure ou égale à 0.1 % en poids et de teneur en hydrogène supérieure ou égale à 12,7% en poids mis en œuvre dans une unité comprenant au moins une zone de craquage pour la charge, une zone de séparation/ stripage des effluents des grains de catalyseur cokés et une zone de régénération des dits grains, **caractérisé en ce qu'**on

(a) disperse au moins un matériau carboné solide à l'état fluidisé, de teneur en carbone supérieure ou égale à 80% en poids, au sein des grains de catalyseur coké,
dans la zone de régénération du catalyseur au sein des grains de catalyseur coké d'un lit dense, la proportion des grains de catalyseur par rapport aux grains de matériau carboné étant constante en tout point de la section de la zone de régénération, cette dernière étant équipée à cet effet d'au moins un garnissage structuré, disposé en amont et/ou en aval de moyens de dispersion du matériau carboné par rapport à la circulation envisagée du catalyseur dans la zone de régénération, ledit garnissage structuré étant formé par un enchevêtrement de plaques, de ruban ou d'ailettes constituant un tamis, ce tamis occupant moins de 10% en surface de la section de la capacité dans laquelle il est placé, mais recouvrant en projection sur celle-ci toute sa surface,
(b) **en ce qu'**on ajuste la quantité de matériau carboné solide à l'état fluidisé dispersée au sein des grains de catalyseur coké pour fournir une quantité de coke Qc supplémentaire sur le catalyseur pour satisfaire l'équation (I) suivante :

$$Q_c = Q_t - Q_i \text{ (I)},$$

où Qi est la teneur en coke initiale sur le catalyseur coké après craquage de la charge et où Qt ou Delta coke est la teneur en coke nécessaire au maintien de la température du catalyseur régénéré et donc au bilan thermique du procédé,

(c) **en ce que** le mélange de grains de catalyseur coké et de matériau carboné solide est brûlé dans la zone de régénération pour produire un catalyseur régénéré appauvri en matériau carboné,

(d) **en ce que** le catalyseur régénéré est mélangé à la charge faiblement cokante dans la zone de craquage pour produire les grains de catalyseur coké et les effluents,

(e) et **en ce que** les grains de catalyseur coké sont séparés des effluents dans la zone de séparation/ stripage puis les grains de catalyseur coké séparés sont envoyés dans la zone de régénération.

2. Procédé selon la revendication 1, **caractérisé en ce que** la totalité du matériau carboné ajouté est brûlée dans la zone de régénération.

3. Procédé selon la revendication 1 ou 2, **caractérisé en ce que** le matériau carboné est fluidisé par tout moyen, dans un effluent liquide ou gazeux non amalgamant avec le matériau carboné, de préférence de l'air.

4. Procédé selon l'une des revendications 1 à 3, **caractérisé en ce que** Qt est choisie variant de 0.5 à 1% en poids lorsque la zone de régénération ne comprend qu'une seule étape, et de 0.8 à 1.45 % en poids pour une combustion partielle dans le premier étage d'une zone de régénération d'un régénérateur multi-étagé, comprenant au moins deux étapes de régénération.

5. Procédé selon l'une des revendications précédentes, **caractérisé en ce que** le matériau carboné est choisi parmi le coke résultant de la cokéfaction du charbon, des cokeurs d'effluents hydrocarbonés de température d'ébullition supérieure à 350°C choisis parmi les coupes lourdes effluents issues de la réaction de craquage principale, HCO (abréviation de « heavy cycle oil ») d'intervalle de distillation compris typiquement entre 360 et 440°C, et slurry d'intervalle de distillation supérieur à 360°C ( noté 360°+), les résidus de biomasse issus de la conversion du bois et/de la cellulose, le charbon broyé dissout dans un hydrocarbure fluide et/ou injecté par soufflage ou pulvérisation, les coupes riches en asphalte provenant d'unités de désalphatage, les cires non valorisables issues de la liquéfaction du charbon par voie indirecte (GTL) ou du procédé Fischer Tropsch de transformation du gaz en hydrocarbures ou un mélange des dites coupes.

6. Procédé selon l'une des revendications précédentes, **caractérisé en ce que** la charge introduite dans la zone de craquage est choisie dans le groupe comprenant les purges d'unité d'hydrocraqueur, ou "bleed", les charges à base coupe gazole de la distillation sous vide de point d'ébullition supérieur à 350°C, et présentant des teneurs en hydrogène supérieures ou égales à 12,7% en poids, les huiles végétales et les hydrocarbures de point d'ébullition inférieur à 160°C, ces charges étant craquées seules ou en mélange dans la zone de craquage principale du procédé.

7. Procédé selon l'une des revendications précédentes, **caractérisé en ce que** le matériau carboné solide à l'état fluidisé contenant le matériau coké est introduit dans la phase dense d'au moins une étape de la zone de régénération lorsqu'elle est multi-étagée.

8. Procédé selon l'une des revendications précédentes, **caractérisé en ce que** la dispersion de matériau carboné solide à l'état fluidisé est obtenue par des moyens de dispersion de celui-ci sur toute la section de la zone de régénération pour que la distribution des grains de catalyseur sur celle du matériau carboné à l'intérieur de la zone de régénération soit voisine de 1.

9. Procédé selon l'une des revendications précédentes, **caractérisé en ce que** la dispersion de matériau carboné solide à l'état fluidisé est effectuée à contrecourant de la circulation de catalyseur, en aval du dit garnissage structuré, celui-ci couvrant tout ou partie de la section de ladite zone de régénération.

10. Procédé selon la revendication 9, **caractérisé en ce que** la dispersion est réalisée en présence d'au moins un garnissage placé dans la phase dense d'un premier étage de la zone de régénération dans le cas d'une zone de régénération multi-étagée.

11. Procédé selon l'une des revendications précédentes, **caractérisé en ce que** le matériau carboné solide à l'état fluidisé est dispersé sur tout ou partie de la hauteur de chaque lit dense de la zone de régénération, chaque dispersion se faisant après homogénéisation du lit fluidisé, ce dernier étant équipé éventuellement d'au moins un garnissage structuré.

12. Dispositif de mise en œuvre du procédé selon l'une des revendications 1 à 11, comprenant au moins un réacteur principal et éventuellement au moins un réacteur secondaire, au moins un désengageur et un stripeur, un régénérateur mono ou multi-étagé, **caractérisé en ce qu'**il comprend des moyens de dispersion homogène d'un matériau carboné à l'état divisé sur des grains de catalyseur cokés issus du désengageur et un strippeur , ces moyens de dispersion homogène étant situés dans le régénérateur lui-même, et **en ce que** le régénérateur est équipé d'au moins un garnissage structuré, disposé en amont et/ou en aval des moyens de dispersion du matériau carboné par rapport à la circulation envisagée du catalyseur dans ledit régénérateur, ledit garnissage structuré étant formé par un enchevêtrement de plaques, de ruban ou d'ailettes constituant un tamis, ce tamis occupant moins de 10% en surface de la section de la capacité dans laquelle il est placé, mais recouvrant en projection sur celle-ci toute sa surface.

13. Dispositif selon la revendication 12, **caractérisé en ce que** les moyens de dispersion sont placés au niveau d'une partie dense du lit fluidisé dans le régénérateur.

14. Dispositif selon l'une des revendications 12 ou 13, **caractérisé en ce que** les moyens de dispersion sont choisis parmi les moyens susceptibles de disperser les mélanges gaz/solides dans un lit dense, de préférence des tubes ouverts et/ou des râteaux formés de plusieurs tubes parallèles s'ouvrant dans le lit dense, ces tubes étant reliés à un tube distributeur .

15. Dispositif selon l'une des revendications 12 à 14, **caractérisé en ce que** le régénérateur est équipé d'au moins un garnissage structuré, disposé en amont des moyens de dispersion du matériau carboné par rapport à la circulation envisagée du catalyseur dans le dit régénérateur au niveau du lit fluidisé dense d'un premier étage de régénération.

## Patentansprüche

1. Verfahren zum katalytischen Cracken einer schwach verkokenden Charge von Conradson-Kohlenstoff von weniger als oder gleich 0,1 Gew.-% und mit einem Wasserstoffgehalt von mehr als oder gleich 12,7 Gew.-%, das in einer Einheit durchgeführt wird, die mindestens einen Crackbereich für die Charge, einen Bereich zum Abtrennen/Strippen der Abströme der verkokten Katalysatorkörner und einen Bereich zur Regeneration der Körner umfasst, **dadurch gekennzeichnet, dass** man

(a) mindestens ein festes kohlenstoffhaltiges Material im fluidisierten Zustand mit einem Kohlenstoffgehalt von mehr als oder gleich 80 Gew.-% in den Körnern von verkoktem Katalysator dispergiert,
wobei im Regenerationsbereich des Katalysators in den Körnern von verkoktem Katalysator einer dichten Schicht das Verhältnis der Katalysatorkörner in Bezug auf die Körner von kohlenstoffhaltigem Material an allen Stellen des Abschnitts des Regenerationsbereichs konstant ist, wobei Letzterer zu diesem Zweck mit mindestens einer strukturierten Packung ausgestattet ist, die stromauf und/oder stromab von Mitteln zur Dispergierung des kohlenstoffhaltigen Materials in Bezug auf die vorgesehene Zirkulation des Katalysators im Regenerationsbereich angeordnet ist, wobei die strukturierte Packung durch ein Gewirr aus Platten, Band oder Rippen gebildet wird, die ein Sieb bilden, wobei dieses Sieb flächenmäßig weniger als 10 % des Abschnitts des Raums einnimmt, in dem es platziert ist, aber in der Projektion darauf seine gesamte Oberfläche bedeckt,
(b) dass man die Menge an festem kohlenstoffhaltigem Material im fluidisierten Zustand, die in den Körnern von verkoktem Katalysator dispergiert wird, so einstellt, dass eine zusätzliche Menge Koks $Q_c$ am Katalysator bereitgestellt wird, um die folgende Gleichung (I) zu erfüllen:

$$Q_c = Q_t - Q_i \ (I),$$

wobei Qi der anfängliche Koksgehalt auf dem verkokten Katalysator nach dem Cracken der Charge ist und wobei Qt oder Delta-Koks der Koksgehalt ist, der zur Aufrechterhaltung der Temperatur des regenerierten Katalysators und somit für die Wärmebilanz des Prozesses erforderlich ist,

(c) dass das Gemisch aus Körnern von verkoktem Katalysator und aus festem kohlenstoffhaltigem Material im Regenerationsbereich abgebrannt wird, um einen regenerierten Katalysator zu erzeugen, der an kohlenstoffhaltigem Material verarmt ist,

(d) dass der regenerierte Katalysator mit der schwach verkokenden Charge im Crackbereich gemischt wird, um die Körner von verkoktem Katalysator und die Abströme zu erzeugen,

(e) und dass die Körner von verkoktem Katalysator von den Abströmen im Abtrenn-/Strippbereich abgetrennt werden und dann die abgetrennten Körner von verkoktem Katalysator in den Regenerationsbereich geleitet werden.

2. Verfahren nach Anspruch 1, **dadurch gekennzeichnet, dass** das gesamte zugegebene kohlenstoffhaltige Material im Regenerationsbereich abgebrannt wird.

3. Verfahren nach Anspruch 1 oder 2, **dadurch gekennzeichnet, dass** das kohlenstoffhaltige Material mit jedem Mittel in einem flüssigen oder gasförmigen Abstrom, der sich nicht mit dem kohlenstoffhaltigen Material vermischt, bevorzugt Luft, fluidisiert wird.

4. Verfahren nach einem der Ansprüche 1 bis 3, **dadurch gekennzeichnet, dass** Qt aus dem Bereich von 0,5 bis 1 Gew.-% ausgewählt ist, wenn der Regenerationsbereich nur einen einzigen Schritt umfasst, und von 0,8 bis 1,45 Gew.-% bei einer partiellen Verbrennung in der ersten Stufe eines Regenerationsbereichs eines mehrstufigen Regenerators, der mindestens zwei Regenerationsschritte umfasst.

5. Verfahren nach einem der vorhergehenden Ansprüche, **dadurch gekennzeichnet, dass** das kohlenstoffhaltige Material ausgewählt ist unter dem Koks, der aus der Verkokung von Kohle resultiert, Kokern von kohlenwasserstoffhaltigen Abströmen mit einer Siedetemperatur über 350 °C, die ausgewählt sind unter schweren Fraktionen von Abströmen aus der Hauptcrackreaktion, HCO (Abkürzung für "Heavy Cycle Oil") mit einem Destillationsintervall von typischerweise zwischen 360 und 440 °C und Slurry mit einem Destillationsintervall über 360 °C (bezeichnet mit 360°+), den Biomasserückständen aus der Umwandlung von Holz und/von Cellulose, zerkleinerter Kohle, die in einem flüssigen Kohlenwasserstoff gelöst ist und/oder durch Blasen oder Sprühen eingespritzt wird, den asphaltreichen Fraktionen aus Entasphaltierungsanlagen, den nicht verwertbaren Wachsen aus der Verflüssigung der Kohle auf indirektem Weg (GTL) oder dem Fischer-Tropsch-Verfahren zur Umwandlung von Gas in Kohlenwasserstoffe oder einem Gemisch der genannten Fraktionen.

6. Verfahren nach einem der vorhergehenden Ansprüche, **dadurch gekennzeichnet, dass** die in den Crackbereich eingeführte Charge aus der Gruppe ausgewählt ist, die die Spülungen einer Hydrocrackereinheit oder "Bleed", die Chargen auf der Basis der Gasölfraktion aus der Vakuumdestillation mit einem Siedepunkt über 350 °C und mit Wasserstoffgehalten von mehr als oder gleich 12,7 Gew.-%, die pflanzlichen Öle und die Kohlenwasserstoffe mit einem Siedepunkt unter 160 °C umfasst, wobei diese Chargen allein oder als Gemisch im Hauptcrackbereich des Verfahrens gecrackt werden.

7. Verfahren nach einem der vorhergehenden Ansprüche, **dadurch gekennzeichnet, dass** das feste kohlenstoffhaltige Material im fluidisierten Zustand, welches das verkokte Material enthält, in die dichte Phase mindestens eines Schritts des Regenerationsbereichs, wenn dieser mehrstufig ist, eingeführt wird.

8. Verfahren nach einem der vorhergehenden Ansprüche, **dadurch gekennzeichnet, dass** die Dispergierung von festem kohlenstoffhaltigem Material im fluidisierten Zustand durch Mittel zu dessen Dispergierung über den gesamten Abschnitt des Regenerationsbereichs erhalten wird, damit die Verteilung der Katalysatorkörner auf der des kohlenstoffhaltigen Materials im Inneren des Regenerationsbereichs nahe bei 1 liegt.

9. Verfahren nach einem der vorhergehenden Ansprüche, **dadurch gekennzeichnet, dass** die Dispergierung von festem kohlenstoffhaltigem Material im fluidisierten Zustand im Gegenstrom zur Katalysatorzirkulation stromab der strukturierten Packung erfolgt, wobei Letztere den gesamten oder einen Teil des Abschnitts des Regenerationsbereichs bedeckt.

10. Verfahren nach Anspruch 9, **dadurch gekennzeichnet, dass** die Dispergierung in Gegenwart mindestens einer Packung ausgeführt wird, die in der dichten Phase einer ersten Stufe des Regenerationsbereichs im Fall eines mehrstufigen Regenerationsbereichs platziert ist.

**11.** Verfahren nach einem der vorhergehenden Ansprüche, **dadurch gekennzeichnet, dass** das feste kohlenstoffhaltige Material im fluidisierten Zustand über die gesamte oder einen Teil der Höhe jeder dichten Schicht des Regenerationsbereichs dispergiert wird, wobei jede Dispergierung nach Homogenisierung der Wirbelschicht erfolgt, wobei Letztere gegebenenfalls mit mindestens einer strukturierten Packung ausgestattet ist.

**12.** Vorrichtung zur Durchführung des Verfahrens nach einem der Ansprüche 1 bis 11, umfassend mindestens einen Hauptreaktor und gegebenenfalls mindestens einen Nebenreaktor, mindestens einen Abtrenner und einen Stripper, einen ein- oder mehrstufigen Regenerator, **dadurch gekennzeichnet, dass** sie Mittel zur homogenen Dispergierung eines kohlenstoffhaltigen Materials im verteilten Zustand auf verkokten Katalysatorkörnern, die aus dem Abtrenner und einem Stripper hervorgegangen sind, umfasst, wobei sich diese Mittel zur homogenen Dispergierung im Regenerator selbst befinden, und dass der Regenerator mit mindestens einer strukturierten Packung ausgestattet ist, die stromauf und/oder stromab der Mittel zur Dispergierung des kohlenstoffhaltigen Materials in Bezug auf die vorgesehene Zirkulation des Katalysators in dem Regenerator angeordnet ist, wobei die strukturierte Packung durch ein Gewirr aus Platten, Band oder Rippen gebildet wird, die ein Sieb bilden, wobei dieses Sieb flächenmäßig weniger als 10 % des Abschnitts des Raums, in dem es platziert ist, einnimmt, aber in Projektion darauf seine gesamte Oberfläche bedeckt.

**13.** Vorrichtung nach Anspruch 12, **dadurch gekennzeichnet, dass** die Dispergierungsmittel auf der Höhe eines dichten Teils der Wirbelschicht im Regenerator angeordnet sind.

**14.** Vorrichtung nach einem der Ansprüche 12 oder 13, **dadurch gekennzeichnet, dass** die Dispergierungsmittel unter Mitteln gewählt sind, die geeignet sind, die Gas/Feststoff-Gemische in einer dichten Schicht zu dispergieren, bevorzugt offenen Rohren und/oder Rechen, die aus mehreren parallelen Rohren gebildet sind, die in die dichte Schicht münden, wobei diese Rohre mit einem Verteilerrohr verbunden sind.

**15.** Vorrichtung nach einem der Ansprüche 12 bis 14, **dadurch gekennzeichnet, dass** der Regenerator mit mindestens einer strukturierten Packung ausgestattet ist, die stromauf der Mittel zur Dispergierung des kohlenstoffhaltigen Materials in Bezug auf die vorgesehene Zirkulation des Katalysators in dem Regenerator auf Höhe der dichten Wirbelschicht einer ersten Regenerationsstufe angeordnet sind.

**Claims**

**1.** Process for the catalytic cracking of a low-coking Conradson carbon feedstock less than or equal to 0.1% by weight and with a hydrogen content greater than or equal to 12.7% by weight implemented in a unit comprising at least one feedstock cracking zone, a zone for separating/stripping the effluents from the coked catalyst grains and a zone for regenerating said grains, **characterised in that**

(a) at least one solid carbonaceous material in the fluidised state, with a carbon content greater than or equal to 80% by weight, is dispersed within the coked catalyst grains,
in the catalyst regeneration zone within the coked catalyst grains of a dense bed, the proportion of the catalyst grains relative to the grains of carbonaceous material being constant at all points of the section of the regeneration zone, the latter being equipped for this purpose with at least one structured packing, arranged upstream and/or downstream of means for dispersing the carbonaceous material with respect to the envisaged circulation of the catalyst in the regeneration zone, said structured packing being formed by an entanglement of plates, strips or fins constituting a sieve, this sieve occupying less than 10% of the surface area of the cross section of the capacity in which it is placed, but covering its entire surface area by projection thereon,
(b) **in that** the amount of solid carbonaceous material in the fluidised state dispersed within the coked catalyst grains is adjusted to provide an additional amount of coke Qc on the catalyst to satisfy the following equation (I):

$$Q_C = Q_t - Q_i \ (I),$$

where Qi is the initial coke content of the coked catalyst after cracking of the feedstock and where Qt or Delta coke is the coke content necessary to maintain the temperature of the regenerated catalyst and therefore the heat balance of the process,
(c) **in that** the mixture of coked catalyst grains and solid carbonaceous material is burnt in the regeneration zone to produce a regenerated catalyst depleted in carbonaceous material,

(d) **in that** the regenerated catalyst is mixed with the low coking feedstock in the cracking zone to produce the coked catalyst grains and the effluents,

(e) and **in that** the coked catalyst grains are separated from the effluents in the separation/stripping zone and then the separated coked catalyst grains are sent to the regeneration zone.

2. Process according to claim 1, **characterised in that** all of the carbonaceous material added is burnt in the regeneration zone.

3. Process according to claim 1 or 2, **characterised in that** the carbonaceous material is fluidised by any means, in a liquid or gaseous effluent not amalgamating with the carbonaceous material, preferably air.

4. Process according to one of claims 1 to 3, **characterised in that** Qt is chosen to vary from 0.5 to 1% by weight when the regeneration zone comprises only one step, and from 0.8 to 1.45% by weight for a partial combustion in the first stage of a regeneration zone of a multi-stage regenerator, comprising at least two regeneration steps.

5. Process according to one of the preceding claims, **characterised in that** the carbonaceous material is chosen from coke resulting from the coking of coal, cokers of hydrocarbon effluents with a boiling point above 350°C chosen from heavy effluent cuts from the main cracking reaction, HCO ("heavy cycle oil") with a distillation range typically between 360 and 440°C, and slurry with a distillation range greater than 360°C (denoted 360°+), biomass residues resulting from the conversion of wood and/or cellulose, crushed charcoal dissolved in a fluid hydrocarbon and/or injected by blowing or spraying, cuts rich in asphalt originating from deasphalting units, non-recoverable waxes from indirect coal liquefaction (GTL) or the Fischer Tropsch process for transforming gas into hydrocarbons or a mixture of said cuts.

6. Process according to one of the preceding claims, **characterised in that** the feedstock introduced into the cracking zone is chosen from the group comprising purges from hydrocracking unit, or "bleed", feedstocks based on gas oil cuts from vacuum distillation with a boiling point greater than 350°C, and having hydrogen contents greater than or equal to 12.7% by weight, vegetable oils and hydrocarbons with a boiling point lower than 160°C, these feedstocks being cracked alone or as a mixture in the main cracking zone of the process.

7. Process according to one of the preceding claims, **characterised in that** the solid carbonaceous material in the fluidised state containing the coked material is introduced into the dense phase of at least one step of the regeneration zone when it is multi-stage.

8. Process according to one of the preceding claims, **characterised in that** the dispersion of solid carbonaceous material in the fluidised state is obtained by means of dispersion thereof over the entire section of the regeneration zone so that the distribution of the grains of catalyst to that of the carbonaceous material inside the regeneration zone is close to 1.

9. Process according to one of the preceding claims, **characterised in that** the dispersion of solid carbonaceous material in the fluidised state is carried out against the circulation of catalyst, downstream of said structured packing, the latter covering all or part of the section of said regeneration zone.

10. Process according to claim 9, **characterised in that** the dispersion is carried out in the presence of at least one packing placed in the dense phase of a first stage of the regeneration zone in the case of a multi-stage regeneration zone.

11. Process according to one of the preceding claims, **characterised in that** the solid carbonaceous material in the fluidised state is dispersed over all or part of the height of each dense bed of the regeneration zone, each dispersion taking place after homogenisation of the fluidised bed, the latter optionally being equipped with at least one structured packing.

12. Device for implementing the process according to one of claims 1 to 11, comprising at least one main reactor and optionally at least one secondary reactor, at least one disengager and one stripper, a single or multi-stage regenerator, **characterised in that** it comprises means of homogeneous dispersion of a carbonaceous material in the divided state on coked catalyst grains from the disengager and a stripper, these means of homogeneous dispersion being located in the regenerator itself, and **in that** the regenerator is equipped with at least one structured packing, arranged upstream and/or downstream of the means for dispersing the carbonaceous material with respect to the envisaged circulation of the catalyst in said regenerator, said structured packing being formed by an entanglement of plates, strips or fins constituting a sieve, this sieve occupying less than 10% of the surface area of the section of the capacity in which it is placed, but covering in projection thereon all of its surface.

13. Device according to claim 12, **characterised in that** the dispersing means are placed at the level of a dense part of the fluidised bed in the regenerator.

14. Device according to one of claims 12 or 13, **characterised in that** the dispersion means are chosen from means capable of dispersing the gas/solid mixtures in a dense bed, preferably open tubes and/or rakes formed from several parallel tubes opening into the dense bed, these tubes being connected to a distributor tube.

15. Device according to one of claims 12 to 14, **characterised in that** the regenerator is equipped with at least one structured packing, arranged upstream of the means for dispersing the carbonaceous material with respect to the envisaged circulation of the catalyst in the said regenerator at the level of the dense fluidised bed of a first regeneration stage.

FIG.1

## RÉFÉRENCES CITÉES DANS LA DESCRIPTION

**Documents brevets cités dans la description**

- US 3092568 A
- US 4875994 A
- US 4828680 A
- EP 719850 A
- US 7022221 B
- US 7077997 B
- WO 200709477L A
- WO 0035575 A
- CN 1763150

**Littérature non-brevet citée dans la description**

- **REZA SADEGHBIGI.** Fluid catalytic cracking handbook. Gulf Professional publishing, 2000